# EUROPEAN PATENT SPECIFICATION

(11) **EP 2 929 517 B1**
(45) Date of publication and mention of the grant of the patent: **08.11.2017**
(21) Application number: 13861744.4
(22) Date of filing: 03.12.2013
(51) Int. Cl.: G08B 21/04, G08B 21/22

(54) **MONITORING SYSTEM AND METHOD**
ÜBERWACHUNGSSYSTEM UND -VERFAHREN
SYSTÈME ET PROCÉDÉ DE CONTRÔLE

(30) Priority: 10.12.2012 FI 20126284
(43) Date of publication of application: 14.10.2015
(73) Proprietor: Seniortek OY, 96300 Rovaniemi (FI)
(72) Inventor: NURMELA, Sami, FI-96300 Rovaniemi (FI); NURMELA, Pasi, FI-96300 Rovaniemi (FI)
(74) Representative: Kolster Oy Ab
(86) International application number: PCT/FI2013/051130
(87) International publication number: WO 2014/091073

(56) References cited:
- EP-A1- 2 068 290
- EP-A1- 2 495 692
- EP-A2- 1 585 078
- EP-A2- 1 585 078
- EP-A2- 2 254 098
- WO-A1-2007/065970
- WO-A1-2011/053900

## Description

### Field

The invention relates to a system and method for monitoring a person in a building.

### Background

Movements of people suffering e.g. from dementia and mental disorders at home, in a hospital building, nursing home, service flat, departments or corresponding premises thereof can be monitored e.g. by means of a wristband to be worn round the wrist and able to be wirelessly connected by radio path with access control base stations attached to a building. A display provided in the system's control room may show a current state in the building, and the system may give an alarm if the person takes the wristband off or tries to leave without permission.

Since any equipment to be attached to a person is inconvenient and it is difficult by means of the above solution to detect e.g. if a person becomes immobile, solutions based on motion detection and pressure detection have been developed. Patent document EP 2068290 presents a social alarm system which comprises a plurality of sensors. Patent document EP 1585078 presents a system and a method for determining whether a resident is at home or away.

However, such a monitoring system presents problems. By motion detectors it is impossible to detect the actual instance of falling, for example, but the assumption of falling is based on detecting no motion. This may cause unnecessary alarms but, in a real problem situation, too slow an alarm. The specifically permitted leaving of a person monitored to an unmonitored area without an alarm at the time monitoring is taking place is impossible. Furthermore, a visit by a attendant to a person monitored may trigger an alarm because in such a situation movement is present in a place where the person monitored himself is not allowed to go.

Thus, a need exists to develop the monitoring system and the related monitoring method.

### Brief description

The object of the invention is to provide an improved solution. This is achieved by an apparatus according to claim 1. The invention also relates to a method according to claim 7.

Preferred embodiments of the invention are disclosed in the dependent claims.

The apparatus and method according to the invention provide several advantages. A representative of the personnel, for example, can visit the room of the person monitored without the monitoring apparatus triggering an alarm. The person monitored also knows that help is available, in case he finds himself in trouble.

### List of figures

The invention will now be described in greater detail in connection with preferred embodiments and with reference to the accompanying drawings, in which
Figure 1 shows an example of a monitoring system and apartments in which the persons monitored and the sensors are located,
Figure 2 shows an example of an apartment that has a toilet,
Figure 3 shows an example of a top and a bottom sensor,
Figure 4 shows an example of the display of the monitoring system, and
Figure 5 shows an example of the system flowchart.

### Description of embodiments

The following embodiments are presented by way of example. Even though the description may refer to "a", "one", or "some" embodiment or embodiments at different points, this does not necessarily mean that each such reference refers to the same embodiment or embodiments or that the feature only applies to one embodiment. Individual features of different embodiments may also be combined to make other embodiments possible.

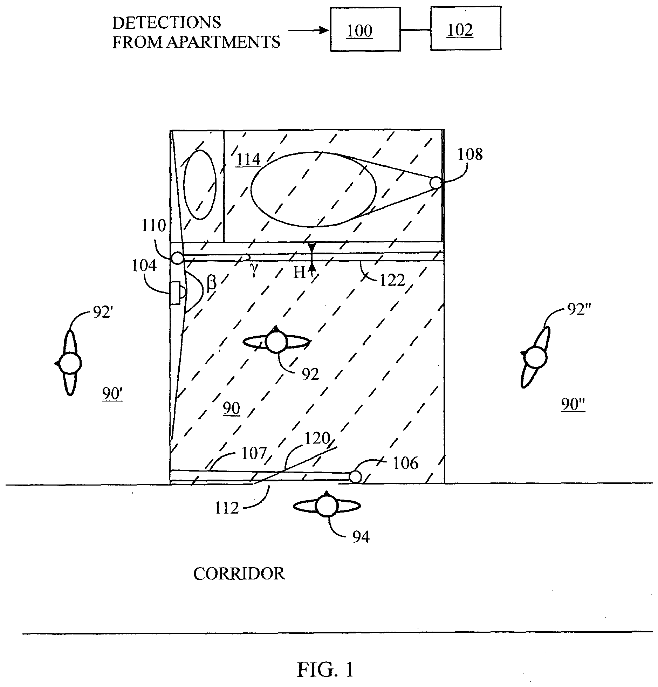

Let us first examine a monitoring system 100 by means of Figure 1. A monitoring system used to monitor a person monitored 92 in apartment 90 is comprised of a control unit 100, an alarm device 102, a room sensor 104, a door sensor 106 and a bed sensor 108. The control unit 100, which receives detected data from sensors 104 - 108, may be comprised of one or more computers with their memories, and one or more computer programs to analyse the detections and to control the alarm device 102. The sensors 104 -108 can be motion detectors.

In an embodiment, the motion detector may be a passive infrared sensor (PIR sensor), which detects infrared radiation generated by body heat. Such a motion detector may detect changes in the intensity of infrared radiation in different parts of the detecting surface of the detector when the person 94, i.e. the object emitting infrared radiation, moves within the area of the beam of the motion detector. In an embodiment, the motion detector is an active infrared detector that detects infrared radiation it sends.

In an embodiment, the motion sensor may be an ultrasound detector, which emits ultrasound e.g. as bursts and receives reflected ultrasound. Such a motion detector is a kind of echo sounder, which can measure the ultrasound growing weaker and/or the time taken by reflection. When changes in these parameters within the area of the beam are detected, it may be interpreted that a moving object, e.g. the person 92, is within the area of the beam.

In an embodiment, the motion detector may be a microwave detector, which emits microwave region electromagnetic radiation but otherwise operates as the ultrasound detector. In an embodiment, the motion detector may also comprise two or more motion detectors of the same or different type. The motion detectors may be known per se, but not of the ways in which they are used according to this application.

A person skilled in the art may, based on his own experience, determine and choose a suitable motion detector for any one purpose. For example, the active infrared detector, ultrasound detector, or microwave detector may be so designed that it establishes a good enough reflection from all clothing materials, and works correctly in different kind of environments.

In an embodiment, the bed sensor 108 can also be, instead of a motion detector, a pressure sensor, which senses the pressure the gravitation of the person 92 causes in the bed 114. The bed sensor 108 can also be on a grounding base or mounted on one or more legs of the bed 114. The pressure sensor may be a capacitive sensor, a piezoelectric sensor, optical sensor, a pressure sensor based on tunnelling, or similar. The pressure sensors may be known per se, but not of the ways in which they are used according to this application. The placement of the pressure sensor in the bed may, however, interfere with the use of the bed, such as making and cleaning the bed. In addition, a large pressure sensor in particular may impede the person's sleep. Different positions of the person may also affect whether or not the person is detected to be in the bed. Further, if an object having the shape and/or weight of a human being is placed in the bed, the system may assume that the person is in the bed even if this is not the case. Therefore, the use of a motion detector to detect presence in the bed 114 may be a better solution, although a pressure sensor as the bed sensor 108 is as such a working solution.

The alarm device 102 may be comprised of one or more displays, one or more loudspeakers, one or more indicator lights, or similar. The alarm device 302 is also known per se, but not of the contexts in which it is used according to this application. The alarm can as such be, for example, a flashing light in the graphic representation on the computer screen, in the apartment where the alarm situation was detected.

In a general case, there may be one or more apartments 90, 90', 90" in the building, and there may be more than one person 92, 92', 92". Normally, each apartment 90, 90', 90" may have one person, only. The building containing the apartments 90, 90', 90" may be the home of a private person, such as the home of the person 92 to be monitored, or a hospital building, a nursing home, a sheltered home, or the like. The apartment 90 may comprise one or more rooms.

The room sensor 104 detects movement in the apartment 90. The room sensor 102 comprises at least one movement detector, which can detect the movement of the person 92 anywhere in the apartment 90 when the person 92 walks or moves about in a wheelchair. In such a case, the room sensor 104 is used to detect movement in other areas than the bed 114. The view to the bed 114 may be blocked by a shade or by otherwise limiting the solid angle. The beam of the room sensor 104 above the bed 114 can extend so that a person lying in the bed 114 is not within the beam. The beam may be at such a height that a person sitting on the bed 114 is not within the beam of the room sensor 104, either.

The movement of the person monitored 92 or another person 94 in the apartment 90 causes one or more detections. Continuous movement may cause several, subsequent movement detections or continuous movement detection. The immobility of the person 92, 94 does not cause movement detection. If only one room sensor is used, it can be mounted on the ceiling, for example, and its aperture β can be approximately 2π steradians (hemispherical solid angle downward). Another option can be to mount one room sensor on a wall, with an aperture of approximately 2π steradians, or as an aperture β = π radians (a planar straight edge) parallel to the floor 304, as shown by Figure 1.

T The bed sensor 108 detects the presence of a person in the bed 114. If the person 92 is somewhere else than the bed 114, the bed sensor 108 does not detect the person 92. The bed sensor 108 can carry out the detection so that the movement or presence in the bed of the person 92 being monitored causes one or more detections. If the person 92 has been detected at least once in the bed 114, and no movement detections originate from the other sensors 104, 106, 108 after that, it will be assumed that the person 92 is in the bed 114 all the time.

T The door sensor 106 detects movement at the door 112. The detected movement may have been caused by the person 92, 94 moving, or by the door 120 moving. The beam 107 of the door sensor 106 may be parallel to the wall, narrow, and small-angled as regards its aperture. The beam 107 of the door sensor 106 can be, for example, less than approximately 50 cm in diameter, and the aperture angle can be less than approximately π/10 (= 18°). The aperture angle can be π/20 or as much as π/100.

The control unit 100 controls the alarm device 102 to give an alarm, if the room sensor 104 does not detect movement in the apartment 90 in a time period longer than a predefined room delay, and if the detections by the door sensor 106 and bed sensor 108 are missing for the time period of said room delay. The room delay may be, for example, 0.5h - 6h, depending on the case. The room delay can be shorter in the daytime than at night. The room delay can be, for example, 3h in the daytime and 1h at night. A different room delay can be set for each apartment 90 and/or for each person 92 being monitored.

The control unit 100 sets a predefined rest delay, which is longer than the room delay, if the bed sensor 108 has detected presence in bed 114. The rest delay can be, for example, 2h - 14h, depending on the case. The rest delay can be of different duration at different times of the day. The rest delay can be 12h at night and 3h in the daytime, for example. During the rest delay, the control unit 100 does not control the alarm device 102 to give an alarm, even though the room sensor 104 does not detect movement in a time period longer than the room delay. The control unit 100 can switch to room delay after the rest delay has ended. Alternatively, the control unit 100 can control the alarm device 102 to give an alarm immediately after the rest delay has lapsed, in case it has taken the room sensor 104 a longer time to detect movement in the apartment 90 than the room delay, in which the room delay has already started during the rest delay.

When the door sensor 106 detects movement by the door 112, the control unit 100 starts, after the previous control delay has ended, a predefined control delay during which the control unit 100 does not control the alarm device 102 to give an alarm. The control delay is shorter than the room delay. The control delay can be 1 minute - 60 minutes, for example.

A situation that does not relate to an event during a rest delay will next be examined. After the control delay, the control unit 100 controls the alarm device 102 to give an alarm, if the bed sensor 104 does not detect movement in the room 90 during the control delay, or the bed sensor 108 does not detect presence in the bed 114. So, the alarm will be given fast, if the person monitored 92 leaves his apartment 92 for a period of time longer than allowed. However, the control delay allows the person monitored 92, for example, to leave the apartment 90 for the time period of the control delay. The reason may be, for example, collecting the mail from the postbox or taking care of another matter outside of the apartment at a fairly anticipated rate. The control unit 100 ends the control delay and returns to room delay, if movement is detected in the apartment 90 during the control delay. Movement will be detected in the apartment 90, if the person monitored 92 returns to the apartment 90 before the control delay runs out completely. If it takes too long to see to a matter supposed to have been handled during the control delay, the reason might be a serious problem. In such a case, help can quickly be sent to the person monitored 92 as a result of the automatic alarm. If, however, the person monitored 92 returns to the apartment during the control delay, monitoring his activity can be commenced in the normal manner by means of the room delay.

A situation that relates to an event during a rest delay will next be examined. During the rest delay, the person monitored 92 has been detected to be in the bed 114. In such a case, the control delay will start if, for example, a representative of the facility's personnel, such as an attendant 94, opens the door 112 to check whether everything is in order in the apartment 90. After the control delay, the control unit 100 controls the alarm device 102 to give an alarm, if the room sensor 104 detects movement in the apartment 90 during the control delay without a new detection by the door sensor 106. If there is no new detection by the door sensor 106, it means that someone came in during the rest delay, for example, at night but did not exit because no new movement took place at the door 112. The reason can be that an attendant 94 has noted a problem and stays in the apartment 90, and because the problem continues, the issue can be difficult for the attendant 94 and/or the person monitored 92, and help is needed, even though the attendant 94 cannot specifically call for help. Such a difficult matter can be violence, for example. The reason can also be that someone not belonging to the apartment 90 has come in and will not exit, which could be a harmful or dangerous situation. The control unit 100 ends the control delay and does not control the alarm device 102 to carry out an alarm, if the door sensor 106 performs movement detection at the door 112 during a control delay taking place within the rest delay. Because the door sensor 106 detects movement at the door 112 during the control delay, it is known that the person 94 who came in the apartment 90 during the initiation of the control delay has left the apartment 90 in connection with the subsequent detection by the door sensor 106.

In an embodiment, the control unit 100 controls, during the rest delay, after the control delay has ended, the alarm device 102 to give an alarm, if the room sensor 104 detects movement in the apartment 90 both during the control delay and after it without motion detection by the door sensor 106.

In an embodiment, the bed sensor 108 comprises a near-zone sensor 110 of the bed 114. The near-zone sensor 110 is able to detect movement in the immediate vicinity of the bed 114, as determined by the location of the beam 122 from the near-zone sensor 110. One or more detections by the near-zone sensor 110 may be caused by the movement of the person monitored 92 or another person 94. Immediate vicinity refers to, for example, the distance of less than a few tens of centimetres between the bed 114 and the beam 122. The beam 122 of the near-zone sensor 110 can be parallel to the edge of the bed 114, narrow, and small-angled as regards its aperture. The beam 122 of the near-zone sensor 110 can be, for example, less than approximately 50 cm in diameter, and the aperture angle γ can be less than approximately π/10 (= 18°). The aperture angle can be π/20 or as much as π/100.

The control unit 100 can set a rest delay, if the near-zone sensor 110 first detects movement near the bed 114 and then the bed sensor 108 detects presence in the bed 114.

In an embodiment, the control unit 100 can set a room delay, if the bed detector 108 first detects presence in the bed 114, and then the near-zone sensor 110 detect movement near the bed 114 before the end of the rest delay.

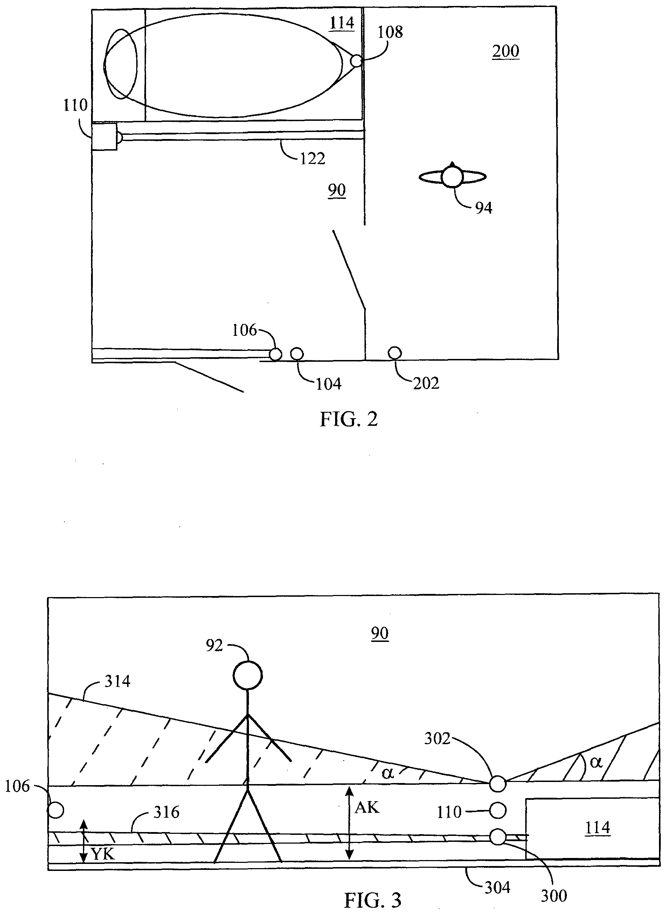

In an embodiment, shown in Figure 2, when the near-zone sensor 110 detects movement during said control delay taking place during said rest delay, the control unit 100 can stop said predefined control delay and set a room delay to make a delayed alarm possible. During a control visit by an attendant 94 or similar, the person monitored 92 can get out of bed 114 and go to the toilet 200, for example, during the room delay.

In an embodiment, when the near-zone sensor 110 detects movement outside of said rest delay during said control delay, the control unit 100 can stop said predefined control delay and enable the room delay for a potential alarm.

In an embodiment, the toilet 200 may be a part of the apartment 90 and the room sensor 104 may comprise a toilet sensor 202 placed in the toilet 200. In such a case, in an embodiment, the movements in the toilet 200 are registered as detections taking place in the apartment 90. The control unit 100 may detect the going to the toilet 200 so that the movement detections from the room sensor 104 end, and movement detections start coming in from the toilet sensor 202 in a shorter time than the room delay. Correspondingly, the control unit 100 may detect the coming out of the toilet 200 so that the movement detections from the toilet sensor 202 end, and movement detections start coming in from the room sensor 104 in a shorter time than the toilet delay.

Alternatively in an embodiment, the movement detections from the toilet sensor 202 can be defined with dedicated delays. The toilet 200 can be defined as a separate area from the apartment 90. The toilet sensor 202 can be positioned in the toilet 200 at the height of approximately one metre, detecting movements from one metre up. The toilet sensor 202 can operate in the similar manner as the room sensor 104. The toilet delay can be set shorter than the room delay, to 5 minutes, for example. The control unit 100 starts, based on the movement detection by the toilet sensor 202, a toilet delay at the end of which the control unit 100 can control the alarm device 102 to carry out an alarm, if detections of movement from all the sensors are missing at the control unit 100 during the toilet delay.

So, if the toilet sensor 202 in an embodiment does not detect any movement at all in the toilet 200 during the toilet delay even though the going in to the toilet 200 has been detected, and neither the door sensor 106 or the room sensor 104 detect movement at the door 112 or the apartment 90 during the toilet delay in question, the control unit 100 will control the alarm device 102 to give an alarm. If the door sensor 106 has detected movement at the door 112 during the toilet delay, and then the room sensor 104 detects movement in the apartment 90, it may be assumed that an attendant 94 has arrived at the apartment 90. In such a case, the control unit 100 can keep the toilet delay by the toilet sensor 202 active as regards the non-detection of the movement.

In an embodiment, the toilet sensor 202 can be the sensor that detects the opening and closing of the toilet 200 door, only. In such a case, it detects that the person monitored 92 goes in the toilet 200 and comes out from there, or does not come out of there. The actual stay in the toilet 200 so that no alarm is carried out is in such a case limited by how long the movement detections of the person monitored 92 by room sensors 104 are missing from the apartment 90. That is, if the person monitored 92 stays in the toilet 200 for a long time, the room delay in the apartment 90 is exceeded without movement detections, and an alarm is given. In this case the toilet delay is the same as the room delay.

In an embodiment, shown in Figure 3, the room sensor 104 may comprise a bottom sensor part 300 for monitoring the movement that takes place lower than a predefined top height YK in the apartment 90 with a beam 316. The room sensor 104 can also comprise a top sensor part 302 for monitoring the movement that takes place higher than a predefined bottom height AK in the apartment 90 with a beam 314. However, the top sensor part 302 does not detect movement occurring at the top of the bed 114 or next to it. Movement that is clearly taking place above the bed 114 can be detected or be undetected by the top sensor part 302. In such a case, the movement takes place higher than where a person sleeping, lying down, reaches. It is possible that the movement of a person sitting on the bed 114 is not detected, either. It is further possible that the reaching of the beam 314 to the parts above the bed 114 is blocked, or that the movement detections made there are not taken into account.

In an embodiment, the control unit 100 can control the alarm device 102 to alarm after a predetermined problem delay has lapsed, if the bottom sensor part 300 detects movement, but the detections from the top sensor part 302 are missing during the problem delay. Alternatively or additionally, in an embodiment, the control unit 100 can control the alarm device 102 to alarm after a predetermined problem delay has lapsed, if the bottom sensor part 300 detects movement, but successive detections from the near-zone sensor 110 and the bed sensor 108 are missing during the problem delay. The control unit 100 can start the problem delay from the first movement detection by the bottom sensor part 300. In an embodiment, the problem delay is shorter than the other delays, such as the control delay, room delay, and rest delay. The problem delay can be, for example, 1 minute - 120 minutes depending on the situation. In many cases, the problem delay can be approximately 5 minutes, for example. This makes it possible for the person monitored 92, who has fallen on the floor 304, to get help quickly, if he cannot get up on his own. The toilet delay can be a delay corresponding to the problem delay.

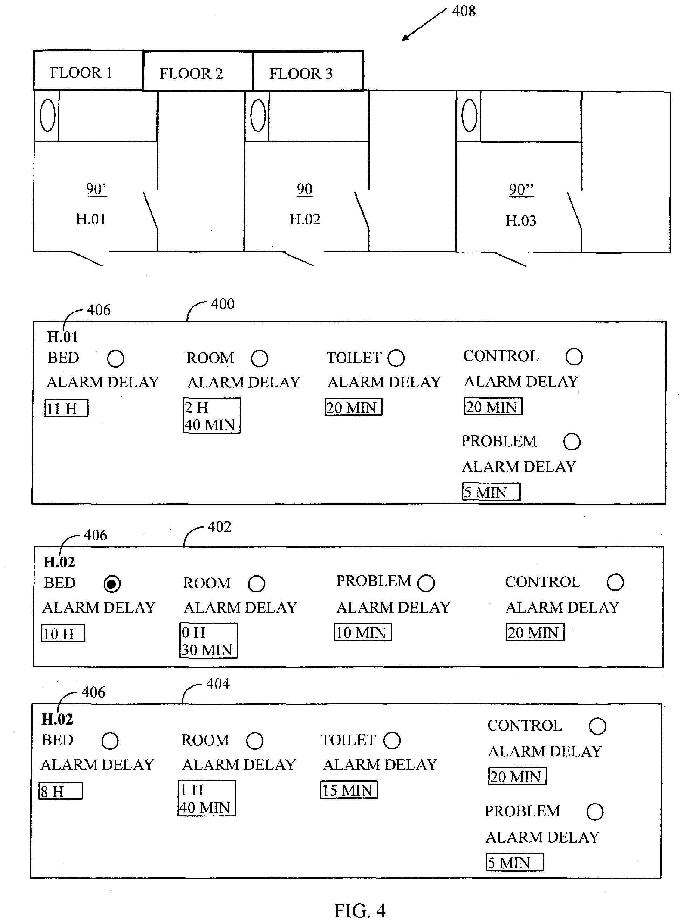

Figure 4 shows an example of the display of the monitoring system in the delay setting state. The display may show windows 400, 402, 404 that have a marking 406 to indicate which apartment 90, 90', 90" the windows 400, 402, 404 concern. Near the windows 400, 402, 404 there may be a floor plan 408 of the apartments 90, 90'. The windows 400, 402, 404 may have menus for the rest delay (bed, alarm delay), room delay (room, alarm delay), control delay (checking, alarm delay), problem delay (problem, alarm delay), and toilet delay (toilet, alarm delay), which can be used by the monitoring user to set the desired delays. A unique delay can be set for each apartment. Different floors of the facility being monitored can be examined on a floor-by-floor basis by selecting the desired floor from the floor menu 408. Each delay can be activated and deactivated. The deactivation can be done by, for example, entering a black dot in the place of the desired alarm delay.

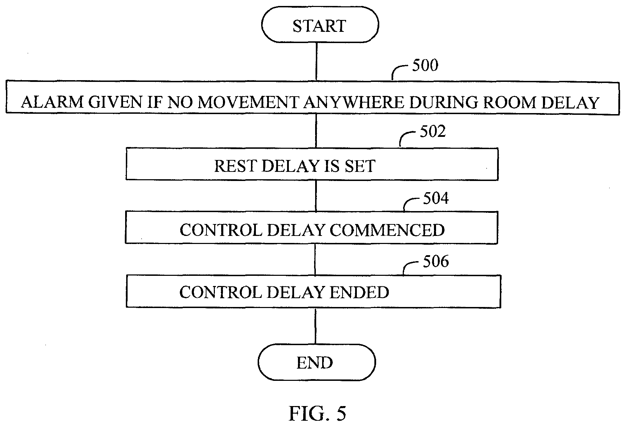

Figure 5 shows an example of the method. In step 500, the control unit 100 controls the alarm device 102 to give an alarm, if the control unit 100 determines that the detections by the room sensor 104 of movement in the apartment 90 are missing in a time period longer than the predefined room delay, and that the detections by the door sensor 106 and bed sensor 108 are missing for the time period of said room delay.

In step 502, the control unit 100 sets a predefined rest delay which is longer than the room delay, if the control unit 100 determines that the room sensor 108 has detected presence in the bed 114, during the rest delay the control unit 100 does not control the alarm device 102 to give an alarm, even though the detections of movement by the room sensor 104 are missing for longer than the room delay, and the control unit 100 shifts to the room delay or to control the alarm device 102 to alarm after the rest delay has lapsed, if a longer period than the room delay has passed from detecting movement in the apartment 90.

In step 504, as the door sensor 106 detects movement by the door 112, the control unit 100 starts, after the previous control delay, a predefined control delay during which the control unit 100 does not control the alarm device 102 to give an alarm, where the control delay is shorter than the room delay, and the control unit 100 controls the alarm device 102 to give an alarm after the control delay has lapsed at times outside of the rest delay, if the control unit 100 determines that the room sensor 104 does not detect movement in the apartment 90 during the control delay, or the bed sensor 108 does not detect presence in the bed 114, and the control unit 100 ends the control delay and starts the room delay, if movement is detected in the apartment 90 during the control delay without detection of movement at the door detector 106 at times outside of the rest delay.

In step 506, the control unit 100 controls, after the control delay has lapsed during the rest delay, the alarm device 102 to alarm if the room sensor 104 has detected movement in the apartment 90 during the control delay without a detection by the door sensor 106, and the control unit 100 ends the control delay, if the door sensor 106 has carried out a detection during a control delay within the rest delay.

When the monitoring system comprises at least one processor and memory having a computer program, the computer program and the memory can, together with said at least one processor, make the monitoring system execute the steps referred to in the method.

The computer program may be placed on a computer program distribution means for the distribution thereof. The computer program distribution means is readable with a data processing device and it may encode the computer program commands to control the operation of the measuring device.

The distribution means, in turn, may be a solution known per se for distributing a computer program, for instance a computer-readable medium, a program storage medium, a computer-readable memory, a computer-readable software distribution package, a computer-readable signal, a computer-readable telecommunication signal or a computer-readable compressed software package.

Even though the invention has been described above with reference to the examples according to the attached drawings, it is clear that the invention is not restricted thereto but may be modified in many ways within the scope of the accompanying claims.

## Claims

1. A monitoring system to monitor a person monitored (92) in an apartment (90), wherein the monitoring system comprises a control unit (100), an alarm device (102), a room sensor (104), a door sensor (106), and a bed sensor (108);
the door sensor (106) is adapted to detect movement by the door (112);
the room sensor (104) is adapted to detect movement in the apartment (90) elsewhere than the bed (114);
the bed sensor (108) is adapted to detect the person's presence in the bed (114);
the control unit (100) is adapted to control the alarm device (102) to give an alarm, if the room sensor (104) does not detect movement in the apartment (90) in a time period longer than a predefined room delay, and if the detections by the door sensor (106) and bed sensor (108) are missing during said room delay;
the control unit (100) is adapted to set a predefined rest delay which is longer than the room delay, if the bed sensor (108) has detected presence in the bed (114), during the rest delay the control unit (100) is adapted not to control the alarm device (102) to give an alarm, even though the room sensor (104) does not detect movement for longer than the room delay, and the control unit (100) is adapted to shift to the room delay or to control the alarm device (102) to give an alarm after the rest delay has lapsed, if it has taken longer than the room delay to detect movement in the apartment (90); and
as the door sensor (106) detects movement by the door (112), the the control unit (100) is adapted, after the previous control delay ends, to start a predefined control delay during which the control unit (100) does not control the alarm device (102) to give an alarm, where the control delay is shorter than the room delay, and after the control delay has lapsed the control unit (100) is, at times outside of the rest delay, adapted to control the alarm device (102) to give an alarm, if the room sensor (104) does not detect movement in the apartment (90) during the control delay, or the bed sensor (108) does not detect presence in the bed (114), and the control unit (100) is adapted to end the control delay and initiate the room delay, if movement is detected in the apartment (90) during the control delay without movement being detected at the door detector (106) at times outside of the rest delay; and
after the control delay has lapsed, the control unit (100) is adapted, during the rest delay, to control the alarm device (102) to give an alarm, if the room sensor (104) has detected movement in the apartment (90) during the control delay without a detection by the door sensor (106), and the control unit (100) is adapted to end the control delay, if the door sensor (106) has performed a detection during the control delay within the rest delay.

2. A system as claimed in claim 1, wherein the room sensor (108) comprises a near-zone sensor (110) for the bed (114), and the control unit (100) is adapted to set a rest delay, if the near-zone sensor (110) has first detected movement near the bed (114) and then the bed sensor (108) has detected presence in the bed (114); the control unit (100) is adapted to set a room delay, if the bed detector (108) has first detected presence in the bed (114) and then the near-zone sensor (110) has detected movement near the bed (114) before the rest delay has lapsed.

3. A system as claimed in claim 1, wherein the control unit (100) is adapted to use a shorter rest delay in the daytime than at night.

4. A system as claimed in claim 1, wherein the room sensor (104) comprises a bottom sensor part (300) to monitor movement in the apartment (90) at a lower level than a predetermined top height (YK), and a top sensor part (302) to monitor movement in the apartment (90) at a higher level than a predetermined bottom height (AK), the top sensor part (302) being adapted not to detect movement above the bed (114) or next to it; and
the control unit (100) is adapted to control the alarm device (102) to give an alarm after a predetermined problem delay has lapsed, the problem delay being a shorter delay than the other delays, if the bottom sensor part (300) has detected movement as the detections by the top sensor part (302) or successive detections by both the near-zone sensor (110) and the bed sensor (108) are missing during the problem delay, where the control unit (100) is adapted to start the problem delay from the first movement detection by the bottom sensor part (300).

5. A system as claimed in claim 1, wherein when the apartment comprises a toilet (200) and when the monitoring system comprises a toilet sensor (202), the control unit (100) is adapted to start, based on a detection by the toilet sensor (202), a toilet delay after which the control unit (100) is adapted to control the alarm device (102) to carry out an alarm, if the detections of movement by all the sensors are missing at the control unit (100) during the toilet delay.

6. A system as claimed in claim 5, wherein if the door sensor (106) has detected movement by the door (112) during the toilet delay and after that the room sensor (104) detects movement in the apartment (90), the control unit (100) is adapted to keep the toilet delay active.

7. A method to monitor a person monitored (92) in an apartment (90), wherein
a control unit (100) controls an alarm device (102) to give an alarm, if the control unit (100) determines that the detections of movement in the apartment (90) by a room sensor (104) are missing during a time period longer than a predetermined room delay, and that the detections by a door sensor (106) and bed sensor (108) are missing during said room delay;
the control unit (100) sets a predetermined rest delay which is longer than the room delay, if the control unit (100) determines that the bed sensor (108) has detected presence in the bed (114), during the rest delay the control unit (100) does not control the alarm device (102) to give an alarm even though the detections of movement by the room sensor (104) are missing for longer than the room delay, and the control unit (100) shifts to room delay, or to control the alarm device (102) to give an alarm after the rest delay has lapsed, if it has taken a longer time than the room delay to detect movement in the apartment (90); and
as the door sensor (106) detects movement by the door (112), the control unit (100) starts, after the previous control delay ends, a predefined control delay during which the control unit (100) does not control the alarm device (102) to give an alarm, where the control delay is shorter than the room delay, and after the control delay has lapsed the control unit (100), at times outside of the rest delay, controls the alarm device (102) to give an alarm, if the control unit (100) determines that the room sensor (104) does not detect movement in the apartment (90) during the control delay, or the bed sensor (108) does not detect presence in the bed (114), and the control unit (100) ends the control delay and initiates the room delay, if movement is detected in the apartment (90) during the control delay without movement being detected at the door detector (106) at times outside of the rest delay; and
the control unit (100) controls after the control delay has lapsed during the rest delay, the alarm device (102) to alarm if the room sensor (104) has detected movement in the apartment (90) during the control delay without a detection by the door sensor (106), and the control unit (100) ends the control delay, if the door sensor (106) has carried out a detection during the control delay within the rest delay.

8. A method as claimed in claim 7, wherein the bed sensor (108) comprises a near-zone sensor (110) for the bed (114), and the control unit (100) sets a rest delay, if the near-zone sensor (110) has first detected movement near the bed (114) and then the bed sensor (108) has detected presence in the bed (114); the control unit (100) sets a room delay, if the bed detector (108) has first detected presence in the bed (114) and then the near-zone sensor (110) has detected movement near the bed (114) before the rest delay has lapsed.

9. A method as claimed in claim 7, wherein the duration of the rest delay is shorter in the daytime than at night.

10. A method as claimed in claim 7, wherein the room sensor (104) comprises a bottom sensor part (300) to monitor movement in the apartment (90) at a lower level than a predetermined top height (YK), and a top sensor part (302) to monitor movement in the apartment (90) at a higher level than a predetermined bottom height (AK), the top sensor part (302) being adapted not to detect movement above the bed (114) or next to it; and
the control unit (100) controls the alarm device (102) to give an alarm after a predetermined problem delay has lapsed, the problem delay being a shorter delay than the other delays, if the bottom sensor part (300) has detected movement as the detections by the top sensor part (302) or successive detections by the near-zone sensor (110) and the bed sensor (108) are missing during the problem delay, where the control unit (100) starts the problem delay from the first movement detection by the bottom sensor part (300).

11. A method as claimed in claim 7, wherein a toilet delay is initiated at the control unit (100), based on a movement detection by the toilet sensor (202), after which the control unit (100) controls the alarm device (102) to carry out an alarm, if the detections by all the sensors are missing at the control unit (100) during the toilet delay.

12. A method as claimed in claim 11, wherein if the door sensor (106) detects movement by the door (112) during the toilet delay and after that the room sensor (104) detects movement in the apartment (90), the control unit (100) keeps the toilet delay active.

13. A control unit as claimed in claim 1, comprising:
at least one processor; and
at least one memory comprising a computer program code,
said at least one memory with said one processor and computer program code being adapted to make the control unit:
control an alarm device (102) to give an alarm, if the control unit (100) determines that the detections of movement in the apartment (90) by a room sensor (104) are missing during a time period longer than a predetermined room delay, and that the detections by a door sensor (106) and bed sensor (108) are missing during said room delay;
set a predetermined rest delay which is longer than the room delay, if the control unit (100) determines that the bed sensor (108) has detected presence in the bed (114), not to control the alarm device (102) to give an alarm during the rest delay even though the detections of movement by the room sensor (104) are missing for longer than the room delay, and shift to room delay, or control the alarm device (102) to give an alarm after the rest delay has lapsed, if it has taken a longer time than the room delay to detect movement in the apartment (90); and
start, as the door sensor (106) detects movement by the door (112) after the end of the previous control delay, a predetermined control delay, and not control the alarm device (102) to give an alarm during said control delay, where the control delay is shorter than the room delay;
control the alarm device (102) to give an alarm after the control delay has lapsed at times outside of the rest delay, if the control unit (100) determines that the room sensor (104) does not detect movement in the apartment (90) during the control delay, or the room sensor (108) does not detect presence in the bed (114);
end the control delay and start the room delay, if movement has been detected in the apartment (90) during the control delay without detection of movement by the door sensor (106) at times outside of the rest delay;
control, after the control delay has lapsed during the rest delay, the alarm device (102) to alarm if the room sensor (104) has detected movement in the apartment (90) during the control delay without a detection by the door sensor (106); and
end the control delay, if the door sensor (106) has carried out a detection during the control delay within the rest delay.

14. A control unit as claimed in claim 13, wherein the bed sensor (108) comprises a near-zone sensor (110) for the bed (114), and said at least one memory with said at least one processor and computer program code are adapted to cause the control unit (100) to set a rest delay, if the near-zone sensor (110) has first detected movement near the bed (114) and then the bed sensor (108) has detected presence in the bed (114); and to set a room delay, if the bed sensor (108) has first detected presence in the bed (114) and then the near-zone sensor (110) has detected movement near the bed (114) before the rest delay has ended.

15. A control unit as claimed in claim 13, wherein said at least one memory with said at least one processor and computer program code are adapted to cause the control unit (100) to use a shorter rest delay in the daytime than at night.

16. A control unit as claimed in claim 13, wherein the room sensor (104) comprises a bottom sensor part (300) to monitor movement in the apartment (90) at a lower level than a predetermined top height (YK), and a top sensor part (302) to monitor movement in the apartment (90) at a higher level than a predetermined bottom height (AK), the top sensor part (302) being adapted not to detect movement above the bed (114) or next to it; and
said at least one memory with said at least one processor and computer code are adapted to cause the control unit (100) to control the alarm device (102) to give an alarm after the predetermined problem delay has lapsed, the problem delay being a shorter delay than the other delays, if the bottom sensor part (300) has detected movement as the detections by the top sensor part (302) or successive detections by both the near-zone sensor (110) and the bed sensor (108) are missing during the problem delay, where said at least one memory with said at least one processor and computer program code are adapted to cause the control unit (100) to start the problem delay from the first movement detection by the bottom sensor part (300).

17. A control unit as claimed in claim 13, wherein said at least one memory with said at least one processor and computer program code are adapted to cause the control unit (100) to start a toilet delay based on a movement detection by a toilet sensor (202), and at the end of the toilet delay to control the alarm device (102) to carry out an alarm, if movement detections by all the sensors are missing at the control unit (100) during the toilet delay.

18. A control unit as claimed in claim 17, wherein if the door sensor (106) has detected movement by the door (112) during the toilet delay and after that the room sensor (104) has detected movement in the apartment (90), said at least one memory with at least one processor and computer program code are adapted to cause the control unit (100) to keep the toilet delay active.

19. A computer program distribution means that is computer-readable, wherein the commands of the computer program are coded on the distribution means to execute the computer program code of claim 13.

## Patentansprüche

1. Überwachungssystem zum Überwachen einer in einer Wohnung (90) überwachten (92) Person, wobei das Überwachungssystem eine Steuerungseinheit (100), eine Alarmvorrichtung (102), einen Raumsensor (104), einen Türsensor (106) und einen Bettsensor (108) umfasst;
wobei der Türsensor (106) dazu angepasst ist, um eine Bewegung durch die Tür (112) festzustellen;
wobei der Raumsensor (104) dazu angepasst ist, um eine Bewegung in der Wohnung (90) anderswo als in dem Bett (114) festzustellen;
wobei der Bettsensor (108) dazu angepasst ist, um die Anwesenheit der Person in dem Bett (114) festzustellen;
wobei die Steuerungseinheit (100) dazu angepasst ist, um die Alarmvorrichtung (102) zu steuern, um einen Alarm auszulösen, wenn der Raumsensor (104) in einer Zeitdauer länger als eine vordefinierte Raumverzögerung keine Bewegung in der Wohnung (90) feststellt und wenn die Feststellungen durch den Türsensor (106) und den Bettsensor (108) während der Raumverzögerung fehlen;
wobei die Steuerungseinheit (100) dazu angepasst ist, um eine vordefinierte Ruheverzögerung festzusetzen, die länger als die Raumverzögerung ist, wenn der Bettsensor (108) eine Anwesenheit in dem Bett (114) festgestellt hat, wobei die Steuerungseinheit (100) während der Ruheverzögerung dazu angepasst ist, um die Alarmvorrichtung (102) nicht zu steuern, um einen Alarm auszulösen, selbst wenn der Raumsensor (104) für länger als die Raumverzögerung keine Bewegung feststellt, und die Steuerungseinheit (100) dazu angepasst ist, um zu der Raumverzögerung zu wechseln oder die Alarmvorrichtung (102) zu steuern, um einen Alarm auszulösen nachdem die Ruheverzögerung abgelaufen ist, wenn es länger als die Raumverzögerung gedauert hat, eine Bewegung in der Wohnung (90) festzustellen; und
wobei, wenn der Türsensor (106) eine Bewegung durch die Tür (112) feststellt, die Steuerungseinheit (100) dazu angepasst ist, um, nachdem die vorherige Steuerungsverzögerung endet, eine vordefinierte Steuerungsverzögerung zu starten, während welcher die Steuerungseinheit (100) die Alarmvorrichtung (102) nicht steuert, um einen Alarm auszulösen, wobei die Steuerungsverzögerung kürzer als die Raumverzögerung ist, und wobei, nachdem die Steuerungsverzögerung abgelaufen ist, die Steuerungseinheit (100) in Zeiten außerhalb der Ruheverzögerung angepasst ist, um die Alarmvorrichtung (102) zu steuern, um einen Alarm auszulösen, wenn der Raumsensor (104) während der Steuerungsverzögerung keine Bewegung in der Wohnung (90) feststellt oder der Bettsensor (108) keine Anwesenheit in dem Bett (114) feststellt, und wobei die Steuerungseinheit (100) dazu angepasst ist, um die Steuerungsverzögerung zu beenden und die Raumverzögerung zu starten, wenn während der Steuerungsverzögerung eine Bewegung in der Wohnung (90) festgestellt wird, ohne dass in Zeiten außerhalb der Ruheverzögerung eine Bewegung an dem Türdetektor (106) festgestellt wird; und
wobei, nachdem die Steuerungsverzögerung abgelaufen ist, die Steuerungseinheit (100) dazu angepasst ist, um während der Ruheverzögerung die Alarmvorrichtung (102) zu steuern, um einen Alarm auszulösen, wenn der Raumsensor (104) während der Steuerungsverzögerung eine Bewegung in der Wohnung (90) festgestellt hat, ohne eine Feststellung durch den Türsensor (106), und wobei die Steuerungseinheit (100) dazu angepasst ist, um die Steuerungsverzögerung zu beenden, wenn der Türsensor (106) während der Steuerungsverzögerung innerhalb der Ruheverzögerung eine Feststellung durchgeführt hat.

2. System nach Anspruch 1, wobei der Raumsensor (108) einen Nahbereichssensor (110) für das Bett (114) umfasst und die Steuerungseinheit (100) dazu angepasst ist, um eine Ruheverzögerung festzusetzen, wenn der Nahbereichssensor (110) zuerst eine Bewegung in der Nähe des Bettes (114) festgestellt hat und dann der Bettsensor (108) eine Anwesenheit in dem Bett (114) festgestellt hat, wobei die Steuerungseinheit (100) dazu angepasst ist, um eine Raumverzögerung festzusetzen, wenn der Bettdetektor (108) zuerst eine Anwesenheit in dem Bett (114) festgestellt hat und dann der Nahbereichssensor (110) eine Bewegung in der Nähe des Bettes (114) festgestellt hat, bevor die Ruheverzögerung abgelaufen ist.

3. System nach Anspruch 1, wobei die Steuerungseinheit (100) dazu angepasst ist, um tagsüber eine kürzere Ruheverzögerung als nachts zu verwenden.

4. System nach Anspruch 1, wobei der Raumsensor (104) einen unteren Sensorteil (300) zum Überwachen einer Bewegung in der Wohnung (90) auf einem niedrigeren Niveau als eine vorbestimmte obere Höhe (YK) und einen oberen Sensorteil (302) zum Überwachen einer Bewegung in der Wohnung (90) auf einem höheren Niveau als eine vorbestimmte untere Höhe (AK) umfasst, wobei der obere Sensorteil (302) dazu angepasst ist, um keine Bewegung oberhalb des Bettes (114) oder daneben festzustellen; und
wobei die Steuerungseinheit (100) dazu angepasst ist, um die Alarmvorrichtung (102) zu steuern, um einen Alarm auszulösen, nachdem eine vorbestimmte Problemverzögerung abgelaufen ist, wobei die Problemverzögerung kürzer als die anderen Verzögerungen ist, wenn der untere Sensorteil (300) eine Bewegung festgestellt hat, während die Feststellungen durch den oberen Sensorteil (302) oder aufeinanderfolgende Feststellungen durch sowohl den Nahbereichssensor (110) als auch den Bettsensor (108) während der Problemverzögerung fehlen, wobei die Steuerungseinheit (100) dazu angepasst ist, um die Problemverzögerung ab der ersten Bewegungsfeststellung durch den unteren Sensorteil (300) zu starten.

5. System nach Anspruch 1, wobei, wenn die Wohnung eine Toilette (200) umfasst und wenn das Überwachungssystem einen Toilettensensor (202) umfasst, die Steuerungseinheit (100) dazu angepasst ist, um basierend auf einer Feststellung durch den Toilettensensor (202) eine Toilettenverzögerung zu starten, wonach die Steuerungseinheit (100) dazu angepasst ist, um die Alarmvorrichtung (102) zu steuern, um einen Alarm durchzuführen, wenn während der Toilettenverzögerung die Bewegungsfeststellungen durch alle Sensoren fehlen.

6. System nach Anspruch 5, wobei, wenn der Türsensor (106) während der Toilettenverzögerung eine Bewegung durch die Tür (112) festgestellt hat und danach der Raumsensor (104) eine Bewegung in der Wohnung (90) feststellt, die Steuerungseinheit (100) dazu angepasst ist, um die Toilettenverzögerung aktiv zu halten.

7. Verfahren zur Überwachung einer in einer Wohnung (90) überwachten (92) Person, wobei
eine Steuerungseinheit (100) eine Alarmvorrichtung (102) steuert, um einen Alarm auszulösen, wenn die Steuerungseinheit (100) bestimmt, dass die Feststellungen von Bewegung in der Wohnung (90) durch einen Raumsensor (104) während einer Zeitdauer länger als eine vorbestimmte Raumverzögerung fehlen, und dass die Feststellungen durch einen Türsensor (106) und Bettsensor (108) während der Raumverzögerung fehlen;
die Steuerungseinheit (100) eine vorbestimmte Ruheverzögerung festsetzt, die länger als die Raumverzögerung ist, wenn die Steuerungseinheit (100) bestimmt, dass der Bettsensor (108) eine Anwesenheit in dem Bett (114) festgestellt hat, wobei die Steuerungseinheit (100) die Alarmvorrichtung (102) während der Ruheverzögerung nicht steuert, um einen Alarm auszulösen, selbst wenn die Feststellungen von Bewegung durch den Raumsensor (104) für länger als die Raumverzögerung fehlen, und die Steuerungseinheit (100) zu der Raumverzögerung oder zu einer Steuerung der Alarmvorrichtung (102), um einen Alarm auszulösen, wechselt, nachdem die Ruheverzögerung abgelaufen ist, wenn es eine längere Zeit als die Raumverzögerung gebraucht hat, um eine Bewegung in der Wohnung (90) festzustellen; und
wenn der Türsensor (106) eine Bewegung durch die Tür (112) feststellt, die Steuerungseinheit (100) nachdem die vorherige Steuerungsverzögerung endet eine vordefinierte Steuerungsverzögerung startet, während der die Steuerungseinheit (100) die Alarmvorrichtung (102) nicht steuert, um einen Alarm auszulösen, wobei die Steuerungsverzögerung kürzer als die Raumverzögerung ist, und nachdem die Steuerungsverzögerung abgelaufen ist steuert die Steuerungseinheit (100) in Zeiten außerhalb der Ruheverzögerung die Alarmvorrichtung (102), um einen Alarm auszulösen, wenn die Steuerungseinheit (100) bestimmt, dass der Raumsensor (104) während der Steuerungsverzögerung keine Bewegung in der Wohnung (90) feststellt oder der Bettsensor (108) keine Anwesenheit in dem Bett (114) feststellt, und die Steuerungseinheit (100) beendet die Steuerungsverzögerung und startet die Raumverzögerung, wenn während der Steuerungsverzögerung eine Bewegung in der Wohnung (90) festgestellt wird, ohne dass eine Bewegung an dem Türdetektor (106) in Zeiten außerhalb der Ruheverzögerung festgestellt wird; und
die Steuerungseinheit (100) nachdem die Steuerungsverzögerung während der Ruheverzögerung abgelaufen ist die Alarmvorrichtung (102) steuert, um einen Alarm auszulösen, wenn der Raumsensor (104) während der Steuerungsverzögerung eine Bewegung in der Wohnung (90) festgestellt hat, ohne eine Feststellung durch den Türsensor (106), und die Steuerungseinheit (100) die Steuerungsverzögerung beendet, wenn der Türsensor (106) während der Steuerungsverzögerung innerhalb der Ruheverzögerung eine Feststellung durchgeführt hat.

8. Verfahren nach Anspruch 7, wobei der Bettsensor (108) eine Nahbereichssensor (110) für das Bett (114) umfasst und die Steuerungseinheit (100) eine Ruheverzögerung festsetzt, wenn der Nahbereichssensor (110) zuerst eine Bewegung in der Nähe des Bettes (114) festgestellt hat und dann der Bettsensor (108) eine Anwesenheit in dem Bett (114) festgestellt hat, wobei die Steuerungseinheit (100) eine Raumverzögerung festsetzt, wenn der Bettdetektor (108) zuerst eine Anwesenheit in dem Bett (114) festgestellt hat und dann der Nahbereichssensor (110) eine Bewegung in der Nähe des Bettes (114) festgestellt hat, bevor die Ruheverzögerung abgelaufen ist.

9. Verfahren nach Anspruch 7, wobei die Dauer der Ruheverzögerung tagsüber kürzer als nachts ist.

10. Verfahren nach Anspruch 7, wobei der Raumsensor (104) einen unteren Sensorteil (300) zum Überwachen einer Bewegung in der Wohnung (90) auf einem niedrigeren Niveau als eine vorbestimmte obere Höhe (YK) und einen oberen Sensorteil (302) zum Überwachen einer Bewegung in der Wohnung (90) auf einem höheren Niveau als eine vorbestimmte untere Höhe (AK) umfasst, wobei der obere Sensorteil (302) dazu angepasst ist, um keine Bewegung oberhalb des Bettes (114) oder daneben festzustellen; und
wobei die Steuerungseinheit (100) die Alarmvorrichtung (102) steuert, um einen Alarm auszulösen, nachdem eine vorbestimmte Problemverzögerung abgelaufen ist, wobei die Problemverzögerung kürzer als die anderen Verzögerungen ist, wenn der untere Sensorteil (300) eine Bewegung festgestellt hat, während die Feststellungen durch den oberen Sensorteil (302) oder aufeinanderfolgende Feststellungen durch den Nahbereichssensor (110) und den Bettsensor (108) während der Problemverzögerung fehlen, wobei die Steuerungseinheit (100) die Problemverzögerung ab der ersten Bewegungsfeststellung durch den unteren Sensorteil (300) startet.

11. Verfahren nach Anspruch 7, wobei an der Steuerungseinheit (100) basierend auf einer Bewegungsfeststellung durch den Toilettensensor (202) eine Toilettenverzögerung gestartet wird, wonach die Steuerungseinheit (100) die Alarmvorrichtung (102) steuert, um einen Alarm durchzuführen, wenn während der Toilettenverzögerung die Feststellungen aller Sensoren an der Steuerungseinheit (100) fehlen.

12. Verfahren nach Anspruch 11, wobei, wenn der Türsensor (106) während der Toilettenverzögerung eine Bewegung durch die Tür (112) feststellt und der Raumsensor (104) danach eine Bewegung in der Wohnung (90) feststellt, die Steuerungseinheit (100) die Toilettenverzögerung aktiv hält.

13. Steuerungseinheit nach Anspruch 1, umfassend:
zumindest einen Prozessor; und
zumindest einen Speicher, der einen Computerprogrammcode umfasst, wobei der zumindest eine Speicher mit dem einen Prozessor und Computerprogrammcode dazu angepasst ist, um zu machen, dass die Steuerungseinheit:
eine Alarmvorrichtung (102) steuert, um einen Alarm auszulösen, wenn die Steuerungseinheit (100) bestimmt, dass die Feststellungen von Bewegung in der Wohnung (90) durch einen Raumsensor (104) während einer Zeitdauer länger als eine vorbestimmte Raumverzögerung fehlen und dass die Feststellungen durch einen Türsensor (106) und Bettsensor (108) während der Raumverzögerung fehlen;
eine vorbestimmte Ruheverzögerung festsetzt, die länger als die Raumverzögerung ist, wenn die Steuerungseinheit (100) bestimmt, dass der Bettsensor (108) eine Anwesenheit in dem Bett (114) festgestellt hat, die Alarmvorrichtung (102) nicht steuert, um einen Alarm auszulösen während der Ruheverzögerung, selbst wenn die Feststellungen von Bewegung durch den Raumsensor (104) für länger als die Raumverzögerung fehlen, und zu einer Raumverzögerung wechselt oder die Alarmvorrichtung (102) steuert, um einen Alarm auszulösen, nachdem die Ruheverzögerung abgelaufen ist, wenn es eine längere Zeit als die Raumverzögerung gedauert hat, eine Bewegung in der Wohnung (90) festzustellen; und
eine vorbestimmte Steuerungsverzögerung startet, wenn der Türsensor (106) nach dem Ende der vorherigen Steuerungsverzögerung eine Bewegung durch die Tür (112) feststellt, und die Alarmvorrichtung (102) nicht steuert, um einem Alarm auszulösen, während der Steuerungsverzögerung, wobei die Steuerungsverzögerung kürzer als die Raumverzögerung ist;
die Alarmvorrichtung (102) steuert, um einen Alarm auszulösen nachdem die Steuerungsverzögerung abgelaufen ist in Zeiten außerhalb der Ruheverzögerung, wenn die Steuerungseinheit (100) bestimmt, dass der Raumsensor (104) während der Steuerungsverzögerung keine Bewegung in der Wohnung (90) feststellt oder der Raumsensor (108) keine Anwesenheit in dem Bett (114) feststellt;
die Steuerungsverzögerung beendet und die Raumverzögerung startet, wenn während der Steuerungsverzögerung eine Bewegung in der Wohnung (90) festgestellt wurde, ohne Feststellung einer Bewegung durch den Türsensor (106) in Zeiten außerhalb der Ruheverzögerung;
die Alarmvorrichtung (102) steuert, nachdem die Steuerungsverzögerung während der Ruheverzögerung abgelaufen ist, um einen Alarm auszulösen, wenn der Raumsensor (104) während der Steuerungsverzögerung eine Bewegung in der Wohnung (90) festgestellt hat, ohne eine Feststellung durch den Türsensor (106); und
die Steuerungsverzögerung beendet, wenn der Türsensor (106) während der Steuerungsverzögerung innerhalb der Ruheverzögerung eine Feststellung durchgeführt hat.

14. Steuerungseinheit nach Anspruch 13, wobei der Bettsensor (108) einen Nahbereichssensor (110) für das Bett (114) umfasst und der zumindest eine Speicher mit dem zumindest einen Prozessor und Computerprogrammproduktcode dazu angepasst sind, um zu bewirken, dass die Steuerungseinheit (100) eine Ruheverzögerung festsetzt, wenn der Nahbereichssensor (110) zuerst eine Bewegung in der Nähe des Bettes (114) festgestellt hat und dann der Bettsensor (108) eine Anwesenheit in dem Bett (114) festgestellt hat, und eine Raumverzögerung festzusetzen, wenn der Bettsensor (108) zuerst eine Anwesenheit in dem Bett (114) festgestellt hat und dann der Nahbereichssensor (110) eine Bewegung in der Nähe des Bettes (114) festgestellt hat, bevor die Ruheverzögerung beendet ist.

15. Steuerungseinheit nach Anspruch 13, wobei der zumindest eine Speicher mit dem zumindest einen Prozessor und Computerprogrammcode dazu angepasst sind, um zu bewirken, dass die Steuerungseinheit (100) tagsüber eine kürzere Ruheverzögerung als nachts verwendet.

16. Steuerungseinheit nach Anspruch 13, wobei der Raumsensor (104) einen unteren Sensorteil (300) zum Überwachen einer Bewegung in der Wohnung (90) auf einem niedrigeren Niveau als eine vorbestimmte obere Höhe (YK) und einen oberen Sensorteil (302) zum Überwachen einer Bewegung in der Wohnung (90) auf einem höheren Niveau als eine vorbestimmte untere Höhe (AK) umfasst, wobei der obere Sensorteil (302) dazu angepasst ist, um eine Bewegung oberhalb des Bettes (114) oder daneben festzustellen; und
wobei der zumindest eine Speicher mit dem zumindest einen Prozessor und Computerprogrammcode dazu angepasst sind, um zu bewirken, dass die Steuerungseinheit (100) die Alarmvorrichtung (102) steuert, um einen Alarm auszulösen, nachdem die vorbestimmte Problemverzögerung abgelaufen ist, wobei die Problemverzögerung eine kürzere Verzögerung als die anderen Verzögerungen ist, wenn der untere Sensorteil (300) eine Bewegung festgestellt hat, während die Feststellungen durch den oberen Sensorteil (302) oder aufeinanderfolgende Feststellungen durch sowohl den Nahbereichssensor (110) als auch den Bettsensor (108) während der Problemverzögerung fehlen, wobei der zumindest eine Speicher mit dem zumindest einen Prozessor und Computerprogrammcode dazu angepasst sind, um zu bewirken, dass die Steuerungseinheit (100) die Problemverzögerung ab der ersten Bewegungsfeststellung durch den unteren Sensorteil (300) startet.

17. Steuerungseinheit nach Anspruch 13, wobei der zumindest eine Speicher mit dem zumindest einen Prozessor und Computerprogrammcode dazu angepasst sind, um zu bewirken, dass die Steuerungseinheit (100) eine Toilettenverzögerung basierend auf einer Bewegungsfeststellung durch einen Toilettensensor (202) startet und an dem Ende der Toilettenverzögerung die Alarmvorrichtung (102) steuert, um einen Alarm durchzuführen, wenn Bewegungsfeststellungen durch alle Sensoren an der Steuerungseinheit (100) während der Toilettenverzögerung fehlen.

18. Steuerungseinheit nach Anspruch 17, wobei, wenn der Türsensor (106) während der Toilettenverzögerung eine Bewegung durch die Tür (112) festgestellt hat und danach der Raumsensor (104) eine Bewegung in der Wohnung (90) festgestellt hat, der zumindest eine Speicher mit zumindest einem Prozessor und Computerprogrammcode dazu angepasst sind, um zu bewirken, dass die Steuerungseinheit (100) die Toilettenverzögerung aktiv hält.

19. Computerprogrammverbreitungsmittel, das computerlesbar ist, wobei die Befehle des Computerprogramms auf dem Verbreitungsmittel codiert sind, um den Computerprogrammcode nach Anspruch 13 auszuführen.

## Revendications

1. Système de contrôle pour contrôler une personne contrôlée (92) dans un appartement (90), dans lequel le système de contrôle comprend une unité de commande (100), un dispositif d'alarme (102), un capteur de chambre (104), un capteur de porte (106) et un capteur de lit (108) ;
le capteur de porte (106) est adapté pour détecter un mouvement par la porte (112) ;
le capteur de chambre (104) est adapté pour détecter un mouvement dans l'appartement (90) ailleurs que dans le lit (114) ;
le capteur de lit (108) est adapté pour détecter la présence de la personne dans le lit (114) ;
l'unité de commande (100) est adaptée pour commander le dispositif d'alarme (102) pour donner une alarme, si le capteur de chambre (104) ne détecte pas de mouvement dans l'appartement (90) dans une période de temps plus longue qu'un délai de chambre prédéfini, et si les détections par le capteur de porte (106) et le capteur de lit (108) manquent pendant ledit délai de chambre ;
l'unité de commande (100) est adaptée pour établir un délai de repos prédéfini qui est plus long que le délai de chambre, si le capteur de lit (108) a détecté une présence dans le lit (114), pendant le délai de repos l'unité de commande (100) est adaptée pour ne pas commander le dispositif d'alarme (102) pour donner une alarme, même si le capteur de chambre (104) ne détecte pas de mouvement pendant un temps plus long que le délai de chambre, et l'unité de commande (100) est adaptée pour passer au délai de chambre ou pour commander le dispositif d'alarme (102) pour donner une alarme après que le délai de repos s'est écoulé, s'il a fallu plus longtemps que le délai de chambre pour détecter un mouvement dans l'appartement (90) ; et
lorsque le capteur de porte (106) détecte un mouvement par la porte (112), l'unité de commande (100) est adaptée, après que le délai de commande précédent s'est achevé, pour démarrer un délai de commande prédéfini pendant lequel l'unité de commande (100) ne commande pas le dispositif d'alarme (102) pour donner une alarme, où le délai de commande est plus court que le délai de chambre, et après que le délai de commande s'est écoulé l'unité de commande (100) est, à des moments situés en dehors du délai de repos, adaptée pour commander le dispositif d'alarme (102) pour donner une alarme, si le capteur de chambre (104) ne détecte pas de mouvement dans l'appartement (90) pendant le délai de commande, ou le capteur de lit (108) ne détecte pas de présence dans le lit (114), et l'unité de commande (100) est adaptée pour achever le délai de commande et entamer le délai de chambre, si un mouvement est détecté dans l'appartement (90) pendant le délai de commande sans qu'un mouvement ne soit détecté au niveau du détecteur de porte (106) à des moments situés en dehors du délai de repos ; et
après que le délai de commande s'est écoulé, l'unité de commande (100) est adaptée, pendant le délai de repos, pour commander le dispositif d'alarme (102) pour donner une alarme, si le capteur de chambre (104) a détecté un mouvement dans l'appartement (90) pendant le délai de commande sans détection par le capteur de porte (106), et l'unité de commande (100) est adaptée pour achever le délai de commande, si le capteur de porte (106) a effectué une détection pendant le délai de commande dans le délai de repos

2. Système selon la revendication 1, dans lequel le capteur de chambre (108) comprend un capteur de zone proche (110) pour le lit (114), et l'unité de commande (100) est adaptée pour établir un délai de repos, si le capteur de zone proche (110) a d'abord détecté un mouvement près du lit (114) et ensuite le capteur de lit (108) a détecté une présence dans le lit (114) ; l'unité de commande (100) est adaptée pour établir un délai de chambre, si le détecteur de lit (108) a d'abord détecté une présence dans le lit (114) et le capteur de zone proche (110) a ensuite détecté un mouvement près du lit (114) avant que le délai de repos se soit écoulé.

3. Système selon la revendication 1, dans lequel l'unité de commande (100) est adaptée pour utiliser un délai de repos plus court dans la journée que la nuit.

4. Système selon la revendication 1, dans lequel le capteur de chambre (104) comprend un élément capteur inférieur (300) pour contrôler un mouvement dans l'appartement (90) à un niveau inférieur à une hauteur maximale prédéterminée (YK), et un élément capteur supérieur (302) pour contrôler un mouvement dans l'appartement (90) à un niveau supérieur à une hauteur minimale prédéterminée (AK), l'élément capteur supérieur (302) étant adapté pour ne pas détecter un mouvement au-dessus du lit (114) ou à côté de celui-ci ; et
l'unité de commande (100) est adaptée pour commander le dispositif d'alarme (102) pour donner une alarme après qu'un délai de problème prédéterminé s'est écoulé, le délai de problème étant un délai plus court que les autres délais, si l'élément capteur inférieur (300) a détecté un mouvement lorsque les détections par l'élément capteur supérieur (302) ou des détections successives à la fois par le capteur de zone proche (110) et par le capteur de lit (108) manquent pendant le délai de problème, où l'unité de commande (100) est adaptée pour démarrer le délai de problème à partir de la première détection de mouvement par l'élément capteur inférieur (300).

5. Système selon la revendication 1 dans lequel lorsque l'appartement comprend des toilettes (200) et lorsque le système de contrôle comprend un capteur de toilettes (202), l'unité de commande (100) est adaptée pour démarrer, sur la base d'une détection par le capteur de toilettes (202), un délai de toilettes après lequel l'unité de commande (100) est adaptée pour commander le dispositif d'alarme (102) pour effectuer une alarme, si les détections de mouvement par tous les capteurs manquent au niveau de l'unité de commande (100) pendant le délai de toilettes.

6. Système selon la revendication 5, dans lequel si le capteur de porte (106) a détecté un mouvement par la porte (112) pendant le délai de toilettes et après cela le capteur de chambre (104) détecte un mouvement dans l'appartement (90), l'unité de commande (100) est adaptée pour maintenir le délai de toilettes actif.

7. Procédé pour contrôler une personne contrôlée (92) dans un appartement (90), dans lequel
une unité de commande (100) commande un dispositif d'alarme (102) pour donner une alarme, si l'unité de commande (100) détermine que les détections de mouvement dans l'appartement (90) par un capteur de chambre (104) manquent pendant une période de temps plus longue qu'un délai de chambre prédéterminé, et que les détections par un capteur de porte (106) et un capteur de lit (108) manquent pendant ledit délai de chambre ;
l'unité de commande (100) établit un délai de repos prédéterminé qui est plus long que le délai de chambre, si l'unité de commande (100) détermine que le capteur de lit (108) a détecté une présence dans le lit (114), pendant le délai de repos l'unité de commande (100) ne commande pas le dispositif d'alarme (102) pour donner une alarme même si les détections de mouvement par le capteur de chambre (104) manquent pendant un temps plus long que le délai de chambre, et l'unité de commande (100) passe au délai de chambre, ou commande le dispositif d'alarme (102) pour donner une alarme après que le délai de repos s'est écoulé, s'il a fallu un temps plus long que le délai de chambre pour détecter un mouvement dans l'appartement (90) ; et
lorsque le capteur de porte (106) détecte un mouvement par la porte (112), l'unité de commande (100) démarre, après que le délai de commande précédent s'est achevé, un délai de commande prédéfini pendant lequel l'unité de commande (100) ne commande pas le dispositif d'alarme (102) pour donner une alarme, où le délai de commande est plus court que le délai de chambre, et après que le délai de commande s'est écoulé l'unité de commande (100), à des moments situés en dehors du délai de repos, commande le dispositif d'alarme (102) pour donner une alarme, si l'unité de commande (100) détermine que le capteur de chambre (104) ne détecte pas de mouvement dans l'appartement (90) pendant le délai de commande, ou le capteur de lit (108) ne détecte pas de présence dans le lit (114), et l'unité de commande (100) achève le délai de commande et entame le délai de chambre, si un mouvement est détecté dans l'appartement (90) pendant le délai de commande sans qu'un mouvement soit détecté au niveau du détecteur de porte (106) à des moments situés en dehors du délai de repos ; et
l'unité de commande (100) commande après que le délai de commande s'est écoulé pendant le délai de repos, le dispositif d'alarme (102) pour produire une alarme si le capteur de chambre (104) a détecté un mouvement dans l'appartement (90) pendant le délai de commande sans détection par le capteur de porte (106), et l'unité de commande (100) achève le délai de commande, si le capteur de porte (106) a effectué une détection pendant le délai de commande dans le délai de repos

8. Procédé selon la revendication 7, dans lequel le capteur de lit (108) comprend un capteur de zone proche (110) pour le lit (114), et l'unité de commande (100) établit un délai de repos, si le capteur de zone proche (110) a d'abord détecté un mouvement près du lit (114) et le capteur de lit (108) a ensuite détecté une présence dans le lit (114) ; l'unité de commande (100) établit un délai de chambre, si le détecteur de lit (108) a d'abord détecté une présence dans le lit (114) et le capteur de zone proche (110) a ensuite détecté un mouvement près du lit (114) avant que le délai de repos se soit écoulé.

9. Procédé selon la revendication 7, dans lequel la durée du délai de repos est plus courte dans la journée que la nuit.

10. Procédé selon la revendication 7, dans lequel le capteur de chambre (104) comprend un élément capteur inférieur (300) pour contrôler un mouvement dans l'appartement (90) à un niveau inférieur à une hauteur maximale prédéterminée (YK), et un élément capteur supérieur (302) pour contrôler un mouvement dans l'appartement (90) à un niveau supérieur à une hauteur minimale prédéterminée (AK), l'élément capteur supérieur (302) étant adapté pour ne pas détecter un mouvement au-dessus du lit (114) ou à côté de celui-ci ; et
l'unité de commande (100) commande le dispositif d'alarme (102) pour donner une alarme après qu'un délai de problème prédéterminé s'est écoulé, le délai de problème étant un délai plus court que les autres délais, si l'élément capteur inférieur (300) a détecté un mouvement lorsque les détections par l'élément capteur supérieur (302) ou des détections successives par le capteur de zone proche (110) et le capteur de lit (108) manquent pendant le délai de problème, où l'unité de commande (100) démarre le délai de problème à partir de la première détection de mouvement par l'élément capteur inférieur (300).

11. Procédé selon la revendication 7, dans lequel un délai de toilettes est entamé au niveau de l'unité de commande (100), sur la base d'une détection de mouvement par le capteur de toilettes (202), après quoi l'unité de commande (100) commande le dispositif d'alarme (102) pour effectuer une alarme, si les détections par tous les capteurs manquent au niveau de l'unité de commande (100) pendant le délai de toilettes.

12. Procédé selon la revendication 11, dans lequel si le capteur de porte (106) détecte un mouvement par la porte (112) pendant le délai de toilettes et après cela le capteur de chambre (104) détecte un mouvement dans l'appartement (90), l'unité de commande (100) maintient le délai de toilettes actif.

13. Unité de commande selon la revendication 1, comprenant :
au moins un processeur ; et
au moins une mémoire comprenant un code de programme informatique,
ladite au moins une mémoire avec ledit un processeur et un code de programme informatique étant adaptés pour amener l'unité de commande à :
commander un dispositif d'alarme (102) pour donner une alarme, si l'unité de commande (100) détermine que les détections de mouvement dans l'appartement (90) par un capteur de chambre (104) manquent pendant une période de temps plus longue qu'un délai de chambre prédéterminé, et que les détections par un capteur de porte (106) et un capteur de lit (108) manquent pendant ledit délai de chambre ;
établir un délai de repos prédéterminé qui est plus long que le délai de chambre, si l'unité de commande (100) détermine que le capteur de lit (108) a détecté une présence dans le lit (114), ne pas commander le dispositif d'alarme (102) pour donner une alarme pendant le délai de repos même si les détections de mouvement par le capteur de chambre (104) manquent pendant un temps plus long que le délai de chambre, et passer au délai de chambre, ou commander le dispositif d'alarme (102) pour donner une alarme après que le délai de repos s'est écoulé, s'il a fallu un temps plus long que le délai de chambre pour détecter un mouvement dans l'appartement (90) ; et
démarrer, lorsque le capteur de porte (106) détecte un mouvement par la porte (112) après la fin du délai de commande précédent, un délai de commande prédéterminé, et ne pas commander le dispositif d'alarme (102) pour donner une alarme pendant ledit délai de commande, où le délai de commande est plus court que le délai de chambre ;
commander le dispositif d'alarme (102) pour donner une alarme après que le délai de commande s'est écoulé à des moments situés en dehors du délai de repos, si l'unité de commande (100) détermine que le capteur de chambre (104) ne détecte pas de mouvement dans l'appartement (90) pendant le délai de commande, ou le capteur de chambre (108) ne détecte pas de présence dans le lit (114) ;
achever le délai de commande et démarrer le délai de chambre, si un mouvement a été détecté dans l'appartement (90) pendant le délai de commande sans détection de mouvement par le capteur de porte (106) à des moments situés en dehors du délai de repos ;
commander, après que le délai de commande s'est écoulé pendant le délai de repos, le dispositif d'alarme (102) pour produire une alarme si le capteur de chambre (104) a détecté un mouvement dans l'appartement (90) pendant le délai de commande sans détection par le capteur de porte (106) ; et
achever le délai de commande, si le capteur de porte (106) a effectué une détection pendant le délai de commande dans le délai de repos.

14. Unité de commande selon la revendication 13, dans laquelle le capteur de lit (108) comprend un capteur de zone proche (110) pour le lit (114), et ladite au moins une mémoire avec ledit au moins un processeur et un code de programme informatique sont adaptés pour amener l'unité de commande (100) à établir un délai de repos, si le capteur de zone proche (110) a d'abord détecté un mouvement près du lit (114) et le capteur de lit (108) a détecté ensuite une présence dans le lit (114) ; et pour établir un délai de chambre, si le capteur de lit (108) a d'abord détecté une présence dans le lit (114) et le capteur de zone proche (110) a ensuite détecté un mouvement près du lit (114) avant que le délai de repos se soit achevé.

15. Unité de commande selon la revendication 13, dans laquelle ladite au moins une mémoire avec ledit au moins un processeur et un code de programme informatique sont adaptés pour amener l'unité de commande (100) à utiliser un délai de repos plus court dans la journée que la nuit.

16. Unité de commande selon la revendication 13, dans laquelle le capteur de chambre (104) comprend un élément capteur inférieur (300) pour contrôler un mouvement dans l'appartement (90) à un niveau inférieur à une hauteur maximale prédéterminée (YK), et un élément capteur supérieur (302) pour contrôler un mouvement dans l'appartement (90) à un niveau supérieur à une hauteur minimale prédéterminée (AK), l'élément capteur supérieur (302) étant adapté pour ne pas détecter un mouvement au-dessus du lit (114) ou à côté de celui-ci ; et
ladite au moins une mémoire avec ledit au moins un processeur et un code informatique sont adaptés pour amener l'unité de commande (100) à commander le dispositif d'alarme (102) pour donner une alarme après que le délai de problème prédéterminé s'est écoulé, le délai de problème étant un délai plus court que les autres délais, si l'élément capteur inférieur (300) a détecté un mouvement lorsque les détections par l'élément capteur supérieur (302) ou des détections successives à la fois par le capteur de zone proche (110) et par le capteur de lit (108) manquent pendant le délai de problème, où ladite au moins une mémoire avec ledit au moins un processeur et un code de programme informatique sont adaptés pour amener l'unité de commande (100) à démarrer le délai de problème à partir de la première détection de mouvement par l'élément capteur inférieur (300).

17. Unité de commande selon la revendication 13, dans laquelle ladite au moins une mémoire avec ledit au moins un processeur et un code de programme informatique sont adaptés pour amener l'unité de commande (100) à démarrer un délai de toilettes sur la base d'une détection de mouvement par un capteur de toilettes (202), et à la fin du délai de toilettes à commander le dispositif d'alarme (102) pour effectuer une alarme, si des détections de mouvement par tous les capteurs manquent au niveau de l'unité de commande (100) pendant le délai de toilettes.

18. Unité de commande selon la revendication 17, dans laquelle si le capteur de porte (106) a détecté un mouvement par la porte (112) pendant le délai de toilettes et après cela le capteur de chambre (104) a détecté un mouvement dans l'appartement (90), ladite au moins une mémoire avec au moins un processeur et un code de programme informatique sont adaptés pour amener l'unité de commande (100) à maintenir le délai de toilettes actif.

19. Moyen de distribution de programme informatique qui est lisible par un ordinateur, dans lequel les commandes du programme informatique sont codées sur le moyen de distribution pour exécuter le code de programme informatique de la revendication 13.
